# EUROPEAN PATENT APPLICATION

(11) **EP 4 578 395 A1**
(43) Date of publication of application: **02.07.2025**
(21) Application number: 23857007.1
(22) Date of filing: 07.07.2023
(51) Int. Cl.: A61B 6/00

(54) **INFORMATION PROCESSING DEVICE, INFORMATION PROCESSING METHOD, AND PROGRAM**

(30) Priority: 26.08.2022 JP 2022135229
(71) Applicant: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2023/025354
(87) International publication number: WO 2024/042890

(57) **Abstract**

An information processing apparatus according to the present disclosure includes: at least one processor, in which the processor is configured to: generate a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and detect a lesion based on at least any one of the low-energy image or the high-energy image and the difference image.

## Description

### BACKGROUND OF THE INVENTION

### 1. Field of the Invention

The present disclosure relates to an information processing apparatus, an information processing method, and a program.

### 2. Description of the Related Art

Contrast-enhanced mammography, which acquires a low-energy image and a high-energy image by performing imaging by irradiating a breast in which a contrast agent is injected with radiation having different energies and generates a difference image representing a difference between the low-energy image and the high-energy image to generate an image in which a lesion or the like is contrast-enhanced, is known. In recent years, since the contrast-enhanced mammography has been included in a comprehensive guideline for breast cancer image diagnosis called a breast imaging reporting and data system (BI-RADS), there is a high possibility that the contrast-enhanced mammography will be widely used as a standard diagnosis method.

However, it is difficult to perform the interpretation of the image obtained by the contrast-enhanced mammography. One of the reasons for the difficulty is an effect of background mammary gland parenchymal enhancement (BPE) due to the contrast agent. The BPE represents a level of enhancement of a normal structure of a mammary gland via the contrast agent, and the visibility of the enhanced lesion greatly varies depending on the level of the BPE. As described above, since the difficulty of the interpretation is high in the contrast-enhanced mammography, it is desired to support even a doctor who is not accustomed to the interpretation so that standard interpretation can be performed.

As a technology related to supporting the interpretation of the image in mammography, for example, in Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>., it is proposed to detect a lesion such as breast cancer by using a Faster Region-based Convolutional Neural Network (R-CNN).

### SUMMARY OF THE INVENTION

In a case in which the lesion detection is performed by using the above-described difference image, it is possible to accurately detect the lesion buried in the mammary gland. However, since the difference image has little information on the mammary gland, it is difficult to accurately determine a mammary gland region. A portion that is contrast-enhanced in a region other than the mammary gland region is likely to be a lesion such as breast cancer, but, in the lesion detection using the difference image having a small amount of information on the mammary gland, the relevance between the mammary gland region and the enhancement region cannot be considered, so that there is a possibility that such a lesion is not detected.

Therefore, even in a case in which the lesion is detected by the Faster R-CNN described in Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>. by using the difference image, it is not possible to sufficiently support the interpretation of the image.

An object of the present disclosed technology is to provide an information processing apparatus, an information processing method, and a program capable of improving support for interpretation of an image generated by contrast-enhanced imaging.

In order to achieve the above-described object, the present disclosure provides an information processing apparatus comprising: at least one processor, in which the processor is configured to: generate a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and detect a lesion based on at least any one of the low-energy image or the high-energy image and the difference image.

It is preferable that the processor is configured to: detect the lesion by combining the difference image and the low-energy image in a channel direction and inputting the combined image to a machine learned model.

It is preferable that the machine learned model includes a channel direction attention mechanism that performs weighting on each channel.

It is preferable that the processor is configured to: detect the lesion based on a first feature value extracted from the difference image and a second feature value extracted from the low-energy image.

It is preferable that the machine learned model includes a first pre-stage operation block, a second pre-stage operation block, and a post-stage operation block, and the processor is configured to: extract the first feature value by inputting the difference image to the first pre-stage operation block; extract the second feature value by inputting the low-energy image to the second pre-stage operation block; and detect the lesion by combining the first feature value and the second feature value in a channel direction and inputting the combined feature value to the post-stage operation block.

It is preferable that the machine learned model includes a channel direction attention mechanism that performs weighting on each channel.

It is preferable that the subject is a breast, and the electromagnetic waves are radiation.

It is preferable that the subject is left and right breasts, the low-energy image includes a first low-energy image and a second low-energy image that are captured by irradiating each of the left and right breasts with radiation having the first energy, the high-energy image includes a first high-energy image and a second high-energy image that are captured by irradiating each of the left and right breasts with radiation having the second energy, and the difference image includes a first difference image representing a difference between the first low-energy image and the first high-energy image and a second difference image representing a difference between the second low-energy image and the second high-energy image.

It is preferable that a first machine learned model includes a first pre-stage operation block and a first post-stage operation block, a second machine learned model includes a second pre-stage operation block and a second post-stage operation block, and the processor is configured to: extract a first feature value by combining the first difference image and the first low-energy image in a channel direction and inputting the combined image to the first pre-stage operation block; extract a second feature value by combining the second difference image and the second low-energy image in the channel direction and inputting the combined image to the second pre-stage operation block; detect the lesion in one of the left breast or the right breast by combining the second feature value with the first feature value and inputting the combined feature value to the first post-stage operation block; and detect the lesion in the other of the left breast or the right breast by combining the first feature value with the second feature value and inputting the combined feature value to the second post-stage operation block.

It is preferable that the processor is configured to: combine the first feature value and the second feature value in the channel direction.

It is preferable that the processor is configured to: combine the first feature value and the second feature value via a cross attention mechanism that generates a weight map representing a degree of relevance between the first feature value and the second feature value and that performs weighting on the first feature value and the second feature value based on the generated weight map.

It is preferable that the machine learned model is generated by training a machine learning model using training data including an input image and a ground-truth image and an augmented image in which a contrast between a lesion region and a non-lesion region in the input image is changed.

The present disclosure provides an information processing method comprising: generating a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and detecting a lesion based on at least any one of the low-energy image or the high-energy image and the difference image.

The present disclosure provides a program causing a computer to execute a process comprising: generating a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and detecting a lesion based on at least any one of the low-energy image or the high-energy image and the difference image.

According to the present disclosed technology, it is possible to provide the information processing apparatus, the information processing method, and the program capable of improving the support for the interpretation of the image generated by the contrast-enhanced imaging.

### BRIEF DESCRIPTION OF THE DRAWINGS

Fig. 1 is a diagram showing an example of an overall configuration of a radiation image capturing system according to a first embodiment.
Fig. 2 is a block diagram showing an example of a configuration of an information processing apparatus.
Fig. 3 is a block diagram showing an example of functions implemented by a control unit of the information processing apparatus.
Fig. 4 is a flowchart schematically showing a flow of contrast-enhanced imaging processing.
Fig. 5 is a flowchart schematically showing a flow of detection processing.
Fig. 6 is a diagram schematically showing lesion detection processing via a lesion detection processing unit.
Fig. 7 is a diagram conceptually showing an example of a configuration of a machine learned model.
Fig. 8 is a diagram showing an example of a channel direction attention mechanism.
Fig. 9 is a diagram schematically showing lesion detection processing according to a second modification example.
Fig. 10 is a diagram conceptually showing a configuration of a machine learned model according to a third modification example.
Fig. 11 is a diagram conceptually showing combination processing of a first feature map and a second feature map.
Fig. 12 is a diagram schematically showing lesion detection processing via a lesion detection processing unit according to a fourth modification example.
Fig. 13 is a diagram schematically showing a modification example of the combination processing.
Fig. 14 is a diagram schematically showing combination processing using a cross attention mechanism.
Fig. 15 is a diagram conceptually showing an example of machine learning processing of a machine learning model.
Fig. 16 is a diagram conceptually showing data augmentation processing.
Fig. 17 is a diagram schematically showing lesion detection processing via a lesion detection processing unit according to a second embodiment.
Fig. 18 is a block diagram showing a configuration of an information processing apparatus according to a modification example of the second embodiment.
Fig. 19 is a block diagram showing a configuration of an information processing apparatus according to another modification example of the second embodiment.

### DESCRIPTION OF THE PREFERRED EMBODIMENTS

Hereinafter, embodiments of the present disclosure will be described in detail with reference to the accompanying drawings.

### [First Embodiment]

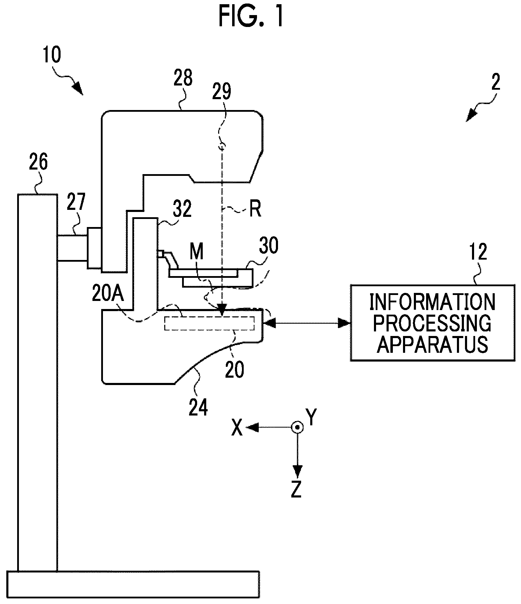

Fig. 1 shows an example of an overall configuration of a radiation image capturing system 2 according to the first embodiment. The radiation image capturing system 2 comprises a mammography apparatus 10 and an information processing apparatus 12. The information processing apparatus 12 is connected to a radiology information system (RIS), a picture archiving and communication system (PACS), and the like (none of which is shown) via a network or the like.

Fig. 1 shows an example of an appearance of the mammography apparatus 10. It should be noted that Fig. 1 shows an example of the appearance in a case in which the mammography apparatus 10 is seen from a left side of a person under an examination.

The mammography apparatus 10 is a radiography apparatus that operates under the control of the information processing apparatus 12 and that irradiates a breast M of a person under an examination, as a subject, with radiation R (for example, X-rays) from a radiation source 29 to capture a radiation image of the breast M. It should be noted that the radiation R is an example of "electromagnetic waves" according to the present disclosed technology.

As shown in Fig. 1, the mammography apparatus 10 comprises an imaging table 24, a base 26, an arm part 28, and a compression unit 32. A radiation detector 20 is disposed inside the imaging table 24. As shown in Fig. 1, in a case in which imaging is performed, in the mammography apparatus 10, the breast M of the person under an examination is positioned on the imaging table 24 by a user, such as a radiologist.

The radiation detector 20 detects the radiation R passing through the breast M as the subject. Specifically, the radiation detector 20 detects the radiation R passing through the breast M of the person under an examination, entering into the imaging table 24, and reaching a detection surface 20A of the radiation detector 20, and generates a radiation image based on the detected radiation R. The radiation detector 20 outputs image data representing the generated radiation image. Hereinafter, the series of operations of irradiating the breast with the radiation R from the radiation source 29 to generate the radiation image via the radiation detector 20 may be referred to as "imaging". The radiation detector 20 may be an indirect conversion type radiation detector that converts the radiation R into light beams and converts the converted light beams into charges, or may be a direct conversion type radiation detector that directly converts the radiation R into charges.

Hereinafter, two directions orthogonal to each other and parallel to the detection surface 20A will be referred to as an X direction and a Y direction. In addition, a direction orthogonal to the X direction and the Y direction will be referred to as a Z direction.

A compression plate 30 that is used for compressing the breast M in a case of performing the imaging is attached to the compression unit 32. The compression plate 30 is moved in a direction approaching or in a direction spaced away from the imaging table 24 by a compression plate drive unit (not shown) provided in the compression unit 32. The compression plate 30 is moved in a direction approaching the imaging table 24 to compress the breast M with the imaging table 24.

The arm part 28 can be rotated with respect to the base 26 by a shaft part 27. The shaft part 27 is fixed to the base 26, and the shaft part 27 and the arm part 28 are rotated integrally. Gears are provided in each of the shaft part 27 and the compression unit 32 of the imaging table 24, and the gears are switched between an engaged state and a non-engaged state, so that a state in which the compression unit 32 of the imaging table 24 and the shaft part 27 are connected to each other and are rotated integrally and a state in which the shaft part 27 is separated from the imaging table 24 and idles can be switched. The elements for switching between transmission and non-transmission of power of the shaft part 27 are not limited to the gears, and various mechanical elements can be used. The arm part 28 and the imaging table 24 can be separately rotated relative to the base 26 with the shaft part 27 as a rotation axis.

The mammography apparatus 10 can perform the imaging on each of the left and right breasts M from a plurality of directions by rotating the arm part 28. For example, it is possible to perform cranio-caudal (CC) imaging and medio-lateral oblique (MLO) imaging.

The radiation image capturing system 2 can perform "contrast-enhanced imaging" in which the imaging is performed in a state in which a contrast agent is injected in the breast M. Specifically, the radiation image capturing system 2 has a contrast enhanced digital mammography (CEDM) function of performing contrast enhancement via energy subtraction.

In the contrast-enhanced imaging, a low-energy image and a high-energy image are acquired by performing the imaging by irradiating the breast M, in which the contrast agent is injected, with the radiation R having different energies. In the present disclosure, a radiation image captured by the radiation R having a first energy will be referred to as a "low-energy image", and a radiation image captured by the radiation R having a second energy higher than the first energy will be referred to as a "high-energy image". Hereinafter, in a case in which the low-energy image and the high-energy image are not distinguished from each other, the low-energy image and the high-energy image will be simply referred to as a radiation image.

In the contrast-enhanced imaging, for example, an iodine contrast agent having a k absorption edge of 32 keV is used as the contrast agent. In the contrast-enhanced imaging in a case in which the iodine contrast agent is used, the first energy need only be set to be lower than the k absorption edge, and the second energy need only be set to be higher than the k absorption edge.

The contrast agent and the body tissue such as the mammary gland are different in absorption characteristics of the radiation R. Therefore, the high-energy image clearly shows the contrast agent in addition to the body tissue such as the mammary gland and the fat. On the other hand, in the low-energy image, the body tissue is clearly shown, but the contrast agent is hardly shown. Therefore, by taking a difference between the low-energy image and the high-energy image, it is possible to generate a difference image in which the mammary gland structure is erased and a lesion or the like stained with the contrast agent is enhanced. The lesion consists of, for example, new cells and is easily stained with the contrast agent.

The mammography apparatus 10 and the information processing apparatus 12 are connected by wired communication or wireless communication. The radiation image generated by the radiation detector 20 in the mammography apparatus 10 is output to the information processing apparatus 12 by wired communication or wireless communication via a communication interface (I/F) (not shown).

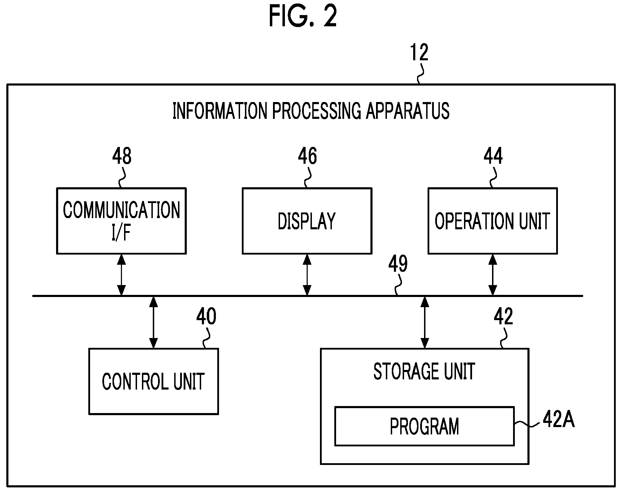

Fig. 2 shows an example of the configuration of the information processing apparatus 12. The information processing apparatus 12 comprises a control unit 40, a storage unit 42, an operation unit 44, a display 46, and a communication I/F 48. The control unit 40, the storage unit 42, the operation unit 44, the display 46, and the communication I/F 48 are connected to each other via a bus 49 such that various kinds of information can be exchanged.

The control unit 40 controls an overall operation of the radiation image capturing system 2. The control unit 40 is configured by, for example, a computer comprising a central processing unit (CPU), a read only memory (ROM), and a random access memory (RAM).

The storage unit 42 stores information related to radiography, the radiation image acquired from the mammography apparatus 10, and the like. In addition, the storage unit 42 stores a program 42A for the control unit 40 to perform various kinds of information processing described later and data for constructing various kinds of machine learned models described later. The storage unit 42 is, for example, a nonvolatile storage device such as a hard disk drive (HDD) or a solid state drive (SSD).

The operation unit 44 includes input devices such as various buttons, switches, a touch panel, a touch pen, and a mouse, which are operated by the user. The display 46 displays information related to the imaging, the radiation image obtained by the imaging, a detection result of the lesion obtained by lesion detection processing described later, and the like.

The communication I/F 48 performs communication of various kinds of data, such as information related to the radiography and the radiation image, with the mammography apparatus 10, the RIS, the PACS, and the like via wired communication or wireless communication.

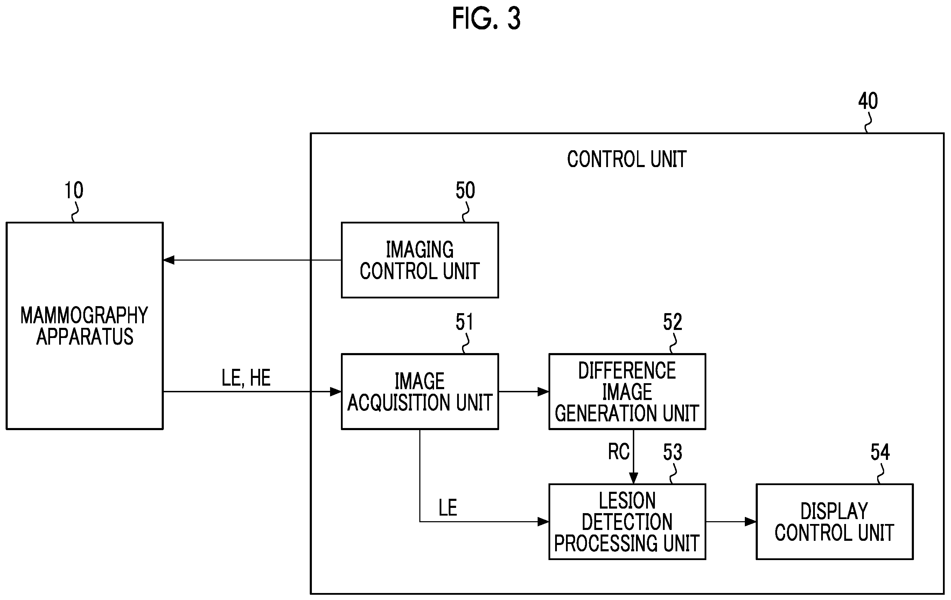

Fig. 3 shows an example of functions implemented by the control unit 40 of the information processing apparatus 12. The control unit 40 implements various functions by executing the processing based on the program 42A stored in the storage unit 42. The control unit 40 functions as an imaging control unit 50, an image acquisition unit 51, a difference image generation unit 52, a lesion detection processing unit 53, and a display control unit 54.

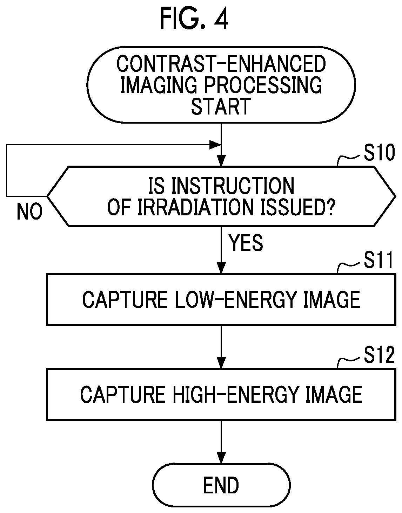

Fig. 4 schematically shows a flow of contrast-enhanced imaging processing. Processing via the imaging control unit 50 will be described with reference to Fig. 4.

First, before the imaging via the mammography apparatus 10 is started, the user, such as the radiologist, injects the contrast agent into the breast M of the person under an examination, positions the breast M in which the contrast agent is injected on the imaging table 24, and compresses the breast M with the compression plate 30.

In step S10, the imaging control unit 50 determines whether or not an instruction of the irradiation with the radiation R is received. In a case in which the instruction of the irradiation is received, the imaging control unit 50 outputs, in step S11, an instruction of the irradiation with the radiation R having the first energy to the mammography apparatus 10. In the mammography apparatus 10, a low-energy image LE is captured by emitting the first energy radiation R toward the breast M.

In next step S12, the imaging control unit 50 outputs an instruction of the irradiation with the radiation R having the second energy to the mammography apparatus 10. In the mammography apparatus 10, a high-energy image HE is captured by emitting the radiation R having the second energy toward the breast M. It should be noted that the high-energy image HE may be captured earlier than the low-energy image LE.

In a case in which the capturing of the low-energy image LE and the high-energy image HE of the breast M ends, the user releases the compression of the breast M for which the imaging ends.

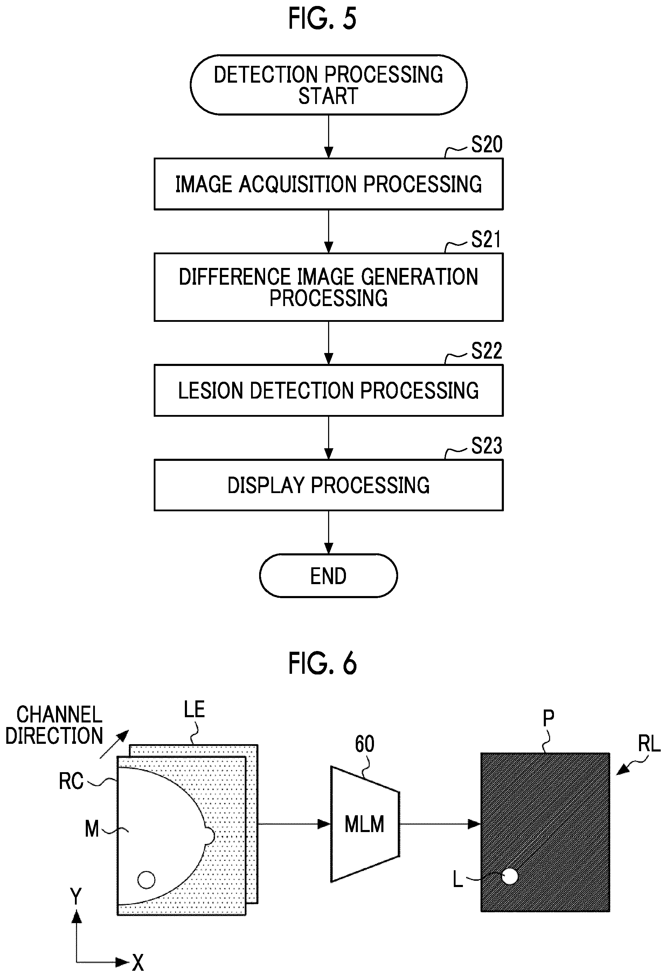

Fig. 5 schematically shows a flow of detection processing. Processing via the image acquisition unit 51, the difference image generation unit 52, the lesion detection processing unit 53, and the display control unit 54 will be described with reference to Fig. 5.

In step S20, the image acquisition unit 51 acquires the low-energy image LE and the high-energy image HE captured by the above-described contrast-enhanced imaging processing.

In next step S21, the difference image generation unit 52 generates a difference image RC representing a difference between the low-energy image LE and the high-energy image HE. For example, the difference image generation unit 52 generates the difference image RC by subtracting an image obtained by multiplying the low-energy image LE by a first weight coefficient from an image obtained by multiplying the high-energy image HE by a second weight coefficient for each corresponding pixel.

In next step S22, the lesion detection processing unit 53 performs the lesion detection processing described later by using the difference image RC and the low-energy image LE.

In next step S23, the display control unit 54 displays the detection result of the lesion obtained by the lesion detection processing on the display 46. The display control unit 54 may display the difference image RC on the display 46 along with the detection result of the lesion.

Fig. 6 schematically shows the lesion detection processing via the lesion detection processing unit 53. The lesion detection processing unit 53 detects a lesion L by inputting the difference image RC and the low-energy image LE to a machine learned model (MLM) 60 that functions as a lesion detection unit. The MLM 60 is configured by a convolutional neural network (CNN). For example, the MLM 60 is configured by fully convolutional networks (FCNs), and outputs an image P in which a region of the lesion L is specified by performing segmentation. For example, in the image P, the region including the lesion L is displayed in a color different from other regions. The image P is a so-called segmentation map. The MLM 60 may be configured by a Segnet, a U-net, or the like that enables semantic segmentation.

For example, the lesion detection processing unit 53 combines the difference image RC and the low-energy image LE in a channel direction, and inputs the combined image to the MLM 60. It should be noted that the combination of the difference image RC and the low-energy image LE in the channel direction means the superimposition of the difference image RC and the low-energy image LE in the channel direction.

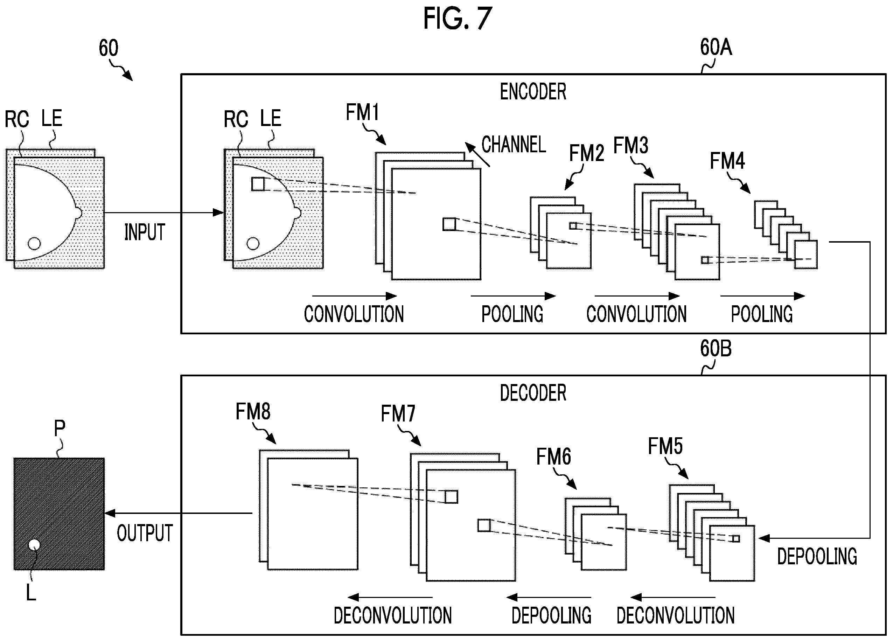

Fig. 7 conceptually shows an example of the configuration of the MLM 60. The MLM 60 includes an encoder 60A and a decoder 60B. The encoder 60A includes a convolutional layer and a pooling layer. The decoder 60B includes a deconvolutional layer and a depooling layer.

The difference image RC and the low-energy image LE combined in the channel direction are input to the encoder 60A. The encoder 60A executes encoding processing, including convolution processing and pooling processing, on the input difference image RC and the low-energy image LE. In the example shown in Fig. 7, the encoder 60A generates a three-channel feature map FM1 by executing the convolution processing on the difference image RC and the low-energy image LE, and generates a three-channel feature map FM2 having a reduced size by executing the pooling processing on the generated feature map FM1. Further, the encoder 60A generates a six-channel feature map FM3 by executing the convolution processing on the feature map FM2, and generates a six-channel feature map FM4 having a reduced size by executing the pooling processing on the generated feature map FM3. The number of channels in each feature map is determined by the number of filters used in a case in which the convolution processing is performed.

The decoder 60B executes decoding processing, including depooling processing and deconvolution processing, on the feature value output from the encoder 60A. **In** the example shown in Fig. 7, the decoder 60B generates a six-channel feature map FM5 having an enlarged size by executing the depooling processing on the feature map FM4 output from the encoder 60A, and generates a three-channel feature map FM6 by executing the deconvolution processing on the generated feature map FM5. Further, the decoder 60B generates a three-channel feature map FM7 having an enlarged size by executing the depooling processing on the feature map FM6, and generates a feature map FM8 having the number of channels of the classes by executing the deconvolution processing on the generated feature map FM7. The number of channels in the feature map is determined by the number of filters used in the deconvolution processing.

For example, the number of classes, which is the number of channels in the feature map FM8, is two classes of whether or not the lesion L is present. The decoder 60B outputs the feature map FM8 as the image P in which the region of the lesion L is specified.

The display control unit 54 displays the difference image RC on the display 46 and displays a marker, such as an arrow for specifying the position of the lesion L specified by the image **P,** in the difference image RC.

The number of convolutional layers, the number of pooling layers, the number of filters used for the convolution processing, and the like included in the encoder 60A can be changed as appropriate. Similarly, the number of deconvolutional layers, the number of depooling layers, the number of filters used in the deconvolution processing, and the like included in the decoder 60B can be changed as appropriate.

As described above, in the present embodiment, the lesion detection processing is performed by using the difference image RC and the low-energy image LE. Since the low-energy image LE includes a large amount of information on the mammary gland than the difference image RC, the low-energy image LE is used in the lesion detection processing in addition to the difference image RC, so that the machine learning and the lesion detection considering the information on the mammary gland can be performed. As a result, it is possible to accurately detect the lesion with a high possibility of being a breast cancer or the like with contrast enhancement in a region other than the mammary gland region. Therefore, according to the present embodiment, the support for the interpretation of the image generated by the contrast-enhanced imaging is improved.

It should be noted that the lesion detection processing unit 53 may perform the lesion detection processing by using the difference image RC and the high-energy image HE instead of the low-energy image LE. In addition, the lesion detection processing unit 53 may perform the lesion detection processing by using the high-energy image HE in addition to the difference image RC and the low-energy image LE. That is, the lesion detection processing unit 53 may combine the difference image RC and the high-energy image HE in the channel direction to input the combined image to the MLM 60, or may combine the difference image RC, the low-energy image LE, and the high-energy image HE in the channel direction to input the combined image to the MLM 60. The present disclosed technology is characterized in that the lesion is detected based on at least any one of the low-energy image LE or the high-energy image HE and the difference image RC.

Hereinafter, various modification examples of the first embodiment will be described.

### [First Modification Example]

The first modification example is different from the above-described embodiment in that the MLM 60 is provided with a channel direction attention mechanism.

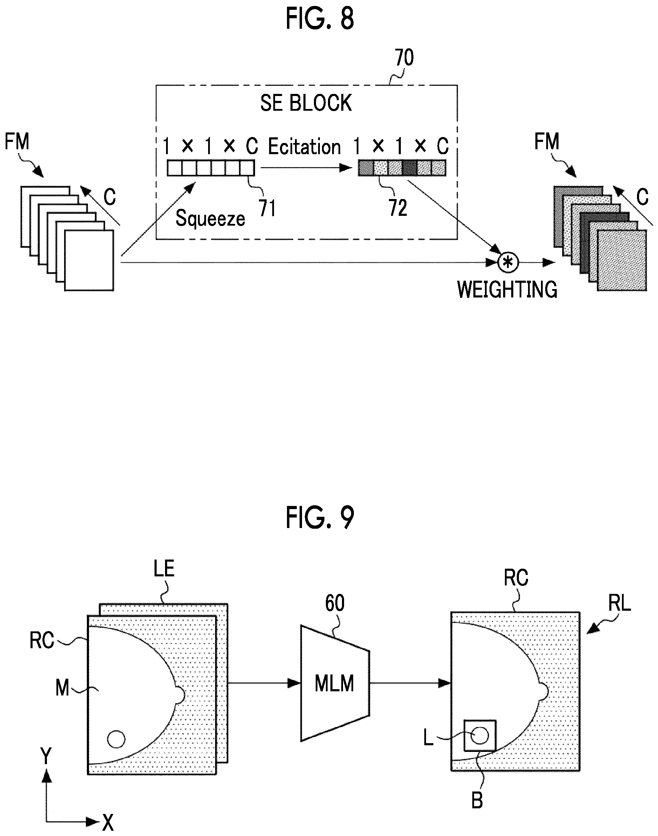

Fig. 8 shows an example of the channel direction attention mechanism. A squeeze- and-excitation (SE) block 70 shown in Fig. 8 is an example of the channel direction attention mechanism, and performs weighting on each channel of one or more feature maps generated inside the MLM 60. The MLM 60 performs processing with more attention to a channel having a large weight than to a channel having a small weight.

The SE block 70 generates an attention mask 72 representing the weight of each channel by executing squeeze processing and excitation processing. First, the SE block 70 performs global pooling processing on the feature map FM and generates a one-dimensional vector 71 having a width and a height of 1 and having the number of channels of C. The number of channels C is the number of channels in the feature map FM. Thereafter, the SE block 70 generates the attention mask 72 by applying two fully connected layers to the one-dimensional vector 71. The attention mask 72 indicates how much each channel should be enhanced.

The MLM 60 performs the weighting on each channel of the feature map FM based on the attention mask 72 generated by the SE block 70. The MLM 60 may apply the SE block 70 to all the feature maps generated by the processing described above, or may apply the SE block 70 to a part of the feature maps.

As in the present modification example, by providing the channel direction attention mechanism in the MLM 60, the machine learning and the lesion detection focusing on a specific channel can be performed, and the accuracy of the lesion detection is improved.

### [Second Modification Example]

The second modification example is different from the above-described embodiment in that the MLM 60 is configured to detect an object instead of performing the segmentation.

Fig. 9 schematically shows the lesion detection processing according to the second modification example. In the present modification example, for example, the MLM 60 is configured by an R-CNN (Regions with CNN features), which is one kind of a CNN, and detects the lesion L as an object.

In the present modification example, the lesion detection processing unit 53 also combines the difference image RC and the low-energy image LE in the channel direction, and inputs the combined image to the MLM 60. In the present modification example, the MLM 60 performs the lesion detection processing of a sliding window type in which a window is slid on the image in which the difference image RC and the low-energy image LE are combined and a patch is cut out from the window to detect the object.

As described above, the MLM 60 detects the lesion L based on the difference image RC and the low-energy image LE, and displays a rectangular bounding box B including the detected lesion L on the difference image RC. The MLM 60 outputs the difference image RC on which the bounding box B is displayed, as the detection result RL of the lesion L. It should be noted that, in the MLM 60, the bounding box B may be displayed on the difference image RC in the low-energy image LE.

In the present modification example, it is possible to provide the MLM 60 with the above-described channel direction attention mechanism. In addition, in the present modification example, the lesion detection processing unit 53 may perform the lesion detection processing by using the difference image RC and the high-energy image HE instead of the low-energy image LE. In addition, the lesion detection processing unit 53 may perform the lesion detection processing by using the high-energy image HE in addition to the difference image RC and the low-energy image LE.

### [Third Modification Example]

The third modification example is different from the above-described embodiment in that the feature value extracted from the difference image RC and the feature value extracted from the low-energy image LE are combined instead of combining the difference image RC and the low-energy image LE.

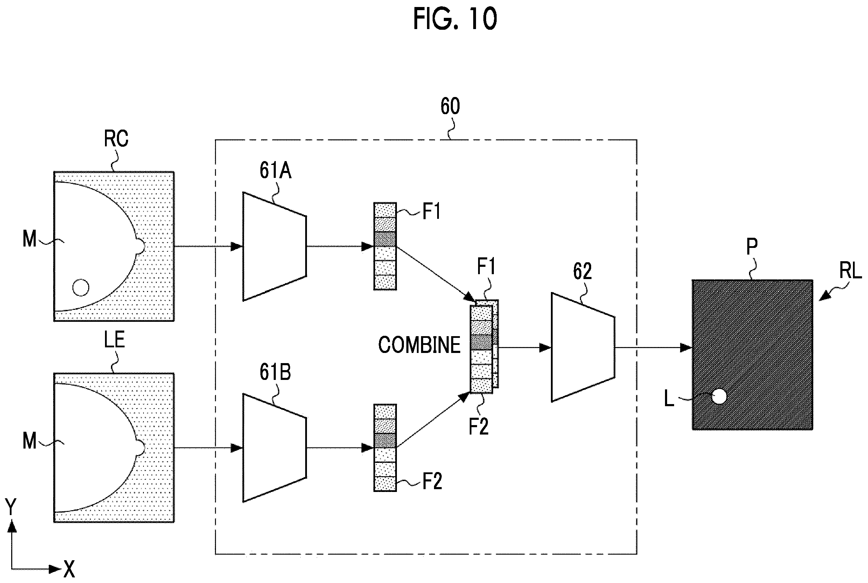

Fig. 10 conceptually shows the configuration of the MLM 60 according to the third modification example. The MLM 60 according to the present modification example includes a first pre-stage operation block 61A, a second pre-stage operation block 61B, and a post-stage operation block 62. The first pre-stage operation block 61A, the second pre-stage operation block 61B, and the post-stage operation block 62 are each configured by a CNN.

In the present modification example, the lesion detection processing unit 53 extracts a first feature map F1 by inputting the difference image RC to the first pre-stage operation block 61A, and extracts a second feature map F2 by inputting the low-energy image LE to the second pre-stage operation block 61B. Then, the lesion detection processing unit 53 combines the first feature map F1 and the second feature map F2 in the channel direction, and inputs the combined feature map to the post-stage operation block 62. The post-stage operation block 62 outputs the image P in which the region of the lesion L is specified by performing the segmentation. It should be noted that the post-stage operation block 62 may be configured to detect the lesion L as the object in the same manner as in the first modification example. The first feature map F1 is an example of a "first feature value" according to the present disclosed technology. The first feature map F2 is an example of a "second feature value" according to the present disclosed technology.

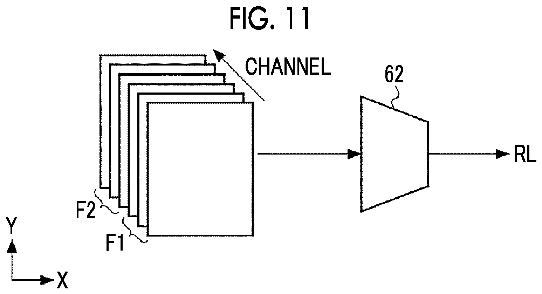

Fig. 11 conceptually shows combination processing of the first feature map F1 and the second feature map F2. The lesion detection processing unit 53 combines the first feature map F1 and the second feature map F2 in a superimposed manner in the channel direction. That is, an overall size of the combined first feature map F1 and second feature map F2 is the same as each size of the first feature map F1 and the second feature map F2, but the number of channels is doubled.

In the present modification example, it is possible to provide the MLM 60 with the above-described channel direction attention mechanism. In the present modification example, the two feature maps are combined in the channel direction, and thus there is a possibility that an effect of the attention in the channel direction is strongly obtained.

It should be noted that, in the present modification example, the feature value extracted from the difference image RC and the feature value extracted from the low-energy image LE are combined, but the feature value extracted from the difference image RC and the feature value extracted from the high-energy image HE may be combined. That is, the high-energy image HE may be input to the second pre-stage operation block 61B instead of the low-energy image LE.

### [Fourth Modification Example]

The fourth modification example shows an example in which the lesion detection processing is performed on the left and right breasts M. In the present modification example, the image acquisition unit 51 acquires the low-energy image LE and the high-energy image HE captured by the contrast-enhanced imaging processing for each of the left and right breasts M. Hereinafter, the low-energy image LE and the high-energy image HE for the left breast M will be referred to as a "first low-energy image LE1" and a "first high-energy image HE1", respectively. In addition, the low-energy image LE and the high-energy image HE for the right breast M will be referred to as a "second low-energy image LE2" and a "second high-energy image HE2", respectively.

In the present modification example, the difference image generation unit 52 generates the difference image RC representing the difference between the low-energy image LE and the high-energy image HE for each of the left and right breasts M. Hereinafter, the difference image RC representing the difference between the first low-energy image LE1 and the first high-energy image HE1 will be referred to as a "first difference image RC1", and the difference image RC representing the difference between the second low-energy image LE2 and the second high-energy image HE2 will be referred to as a "second difference image RC2".

In the present modification example, the lesion detection processing unit 53 detects the lesion from the left breast M by using the first difference image RC1 and the first low-energy image LE1. In addition, the lesion detection processing unit 53 detects the lesion from the right breast M by using the second difference image RC2 and the second low-energy image LE2.

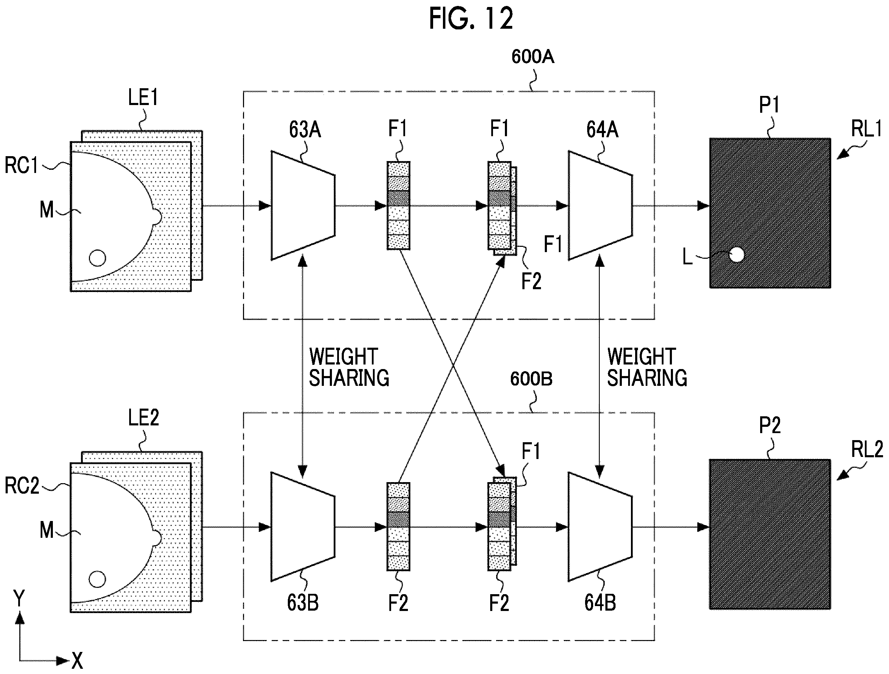

Fig. 12 schematically shows the lesion detection processing via the lesion detection processing unit 53 according to the fourth modification example. The first MLM 600A includes a first pre-stage operation block 63A and a first post-stage operation block 64A. The second MLM 600B includes a second pre-stage operation block 63B and a second post-stage operation block 64B. The lesion detection processing unit 53 combines the first difference image RC1 and the first low-energy image LE1 in the channel direction to input the combined image to the first MLM 600A, and combines the second difference image RC2 and the second low-energy image LE2 in the channel direction to input the combined image to the second MLM 600B.

The first pre-stage operation block 63A outputs the first feature map F1 generated by performing the convolution processing. The second pre-stage operation block 63B outputs the second feature map F2 generated by performing the convolution processing. The lesion detection processing unit 53 combines the second feature map F2 with the first feature map F1 to input the combined feature map to the first post-stage operation block 64A, and combines the first feature map F1 with the second feature map F2 to input the combined feature map to the second post-stage operation block 64B. In the present modification example, the lesion detection processing unit 53 combines the first feature map F1 with the second feature map F2 in the channel direction.

The first pre-stage operation block 63A and the second pre-stage operation block 63B have the same configuration, and share the weights of the filters. The first post-stage operation block 64A and the second post-stage operation block 64B have the same configuration, and share the weights of the filters.

The first post-stage operation block 64A performs the segmentation based on the combined first feature map F1 and second feature map F2 and outputs the image P1 in which the region of the lesion L of the left breast M is specified, as a detection result RL1. Similarly, the second post-stage operation block 64B performs the segmentation based on the combined first feature map F1 and second feature map F2 and outputs the image P2 in which the region of the lesion L of the right breast M is specified, as a detection result RL2.

In the present modification example, since the first feature map F1 and the second feature map F2 are combined with each other in a cross manner, the machine learning and the lesion detection considering the symmetry of the left and right breasts M can be performed. For example, a lesion candidate detected from only one of the left breast M or the right breast M is likely to be the lesion. In the present modification example, it is possible to accurately detect lesion candidates or the like that are asymmetric between the left and right sides as the lesion.

It should be noted that, in the present modification example, the first difference image RC1 and the first low-energy image LE1 are combined and input to the first MLM 600A, but the first difference image RC1 and the first high-energy image HE1 may be combined and input to the first MLM 60A. Further, in the present modification example, the second difference image RC2 and the second low-energy image LE2 are combined and input to the second MLM 60B, but the second difference image RC2 and the second high-energy image HE2 may be combined and input to the second MLM 600B.

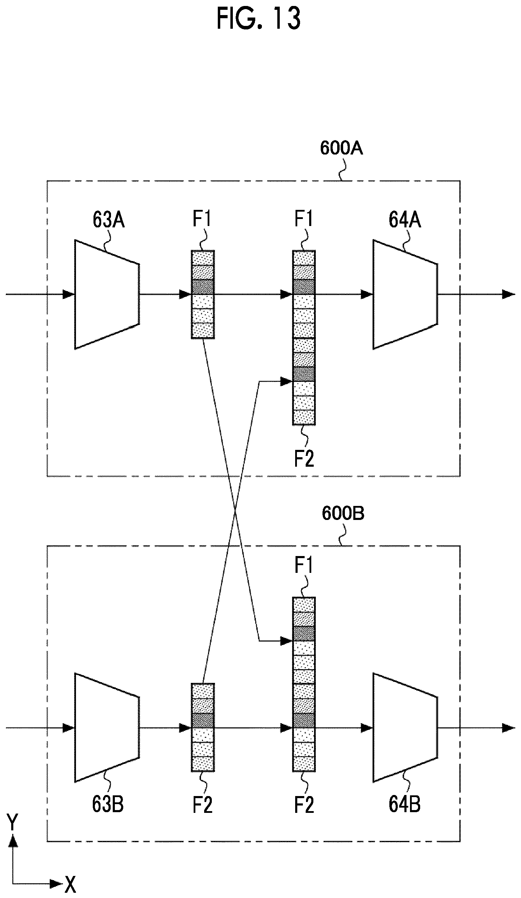

In addition, in the present modification example, although the lesion detection processing unit 53 combines the first feature map F1 and the second feature map F2 in the channel direction, as shown in Fig. 13, the first feature map F1 and the second feature map F2 may be combined in a row direction or a column direction (that is, the X direction or the Y direction).

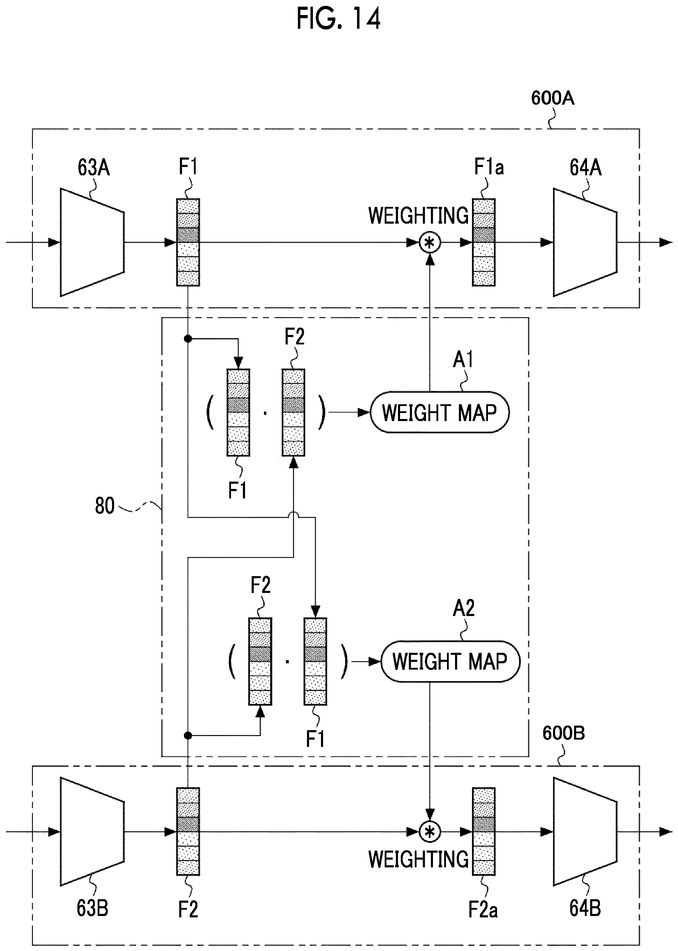

In addition, as shown in Fig. 14, the lesion detection processing unit 53 may combine the first feature map F1 and the second feature map F2 via a cross attention mechanism 80. The cross attention mechanism 80 performs a matrix operation of multiplying the first feature map F1 by the second feature map F2 to calculate a weight map A1, and performs a matrix operation of multiplying the second feature map F2 by the first feature map F1 to calculate a weight map A2. Each of the weight map A1 and the weight map A2 represents a degree of relevance between the first feature map F1 and the second feature map F2.

The cross attention mechanism 80 performs weighting of the first feature map F1 based on the weight map A1, and performs weighting of the second feature map F2 based on the weight map A2. A first feature map F1a subjected to the weighting is input to the first post-stage operation block 64A. The second feature map F2a subjected to the weighting is input to the second post-stage operation block 64B.

### [Machine Learning Processing]

Next, an example of machine learning processing for generating the MLM 60 will be described.

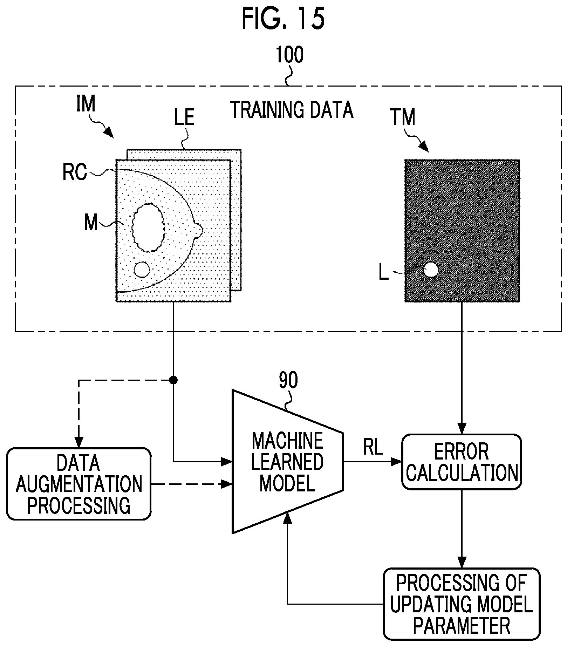

Fig. 15 conceptually shows an example of the machine learning processing of a machine learning model 90. As shown in Fig. 15, the MLM 60 is generated by training the machine learning model 90 through the machine learning using training data 100 in the training phase. For example, the training data 100 includes a combination of the input image IM and the ground-truth image TM. A large number of input images IM and ground-truth images TM are used in the learning processing of the machine learning model 90. The input image IM is, for example, an image in which the difference image RC and the low-energy image LE are combined in the channel direction. The ground-truth image TM is an image in which a region of a true lesion L is specified.

The machine learning model 90 is trained through the machine learning by using, for example, an error back propagation method. In the training phase, an error calculation between the image obtained by inputting the input image IM to the machine learning model 90 and the ground-truth image TM and processing of updating a model parameter of the machine learning model 90 based on a result of the error calculation are repeatedly performed. The model parameter includes the weight of the filter and the like.

Further, in the training phase, data augmentation processing is performed. In the data augmentation processing, an augmented image AM in which a contrast between the region including the lesion L (hereinafter, referred to as a lesion region) and other regions (hereinafter, referred to as a non-lesion region) is changed is generated based on the input image IM. Then, the machine learning of the machine learning model 90 is performed by using the augmented image AM generated by the data augmentation processing and the ground-truth image TM.

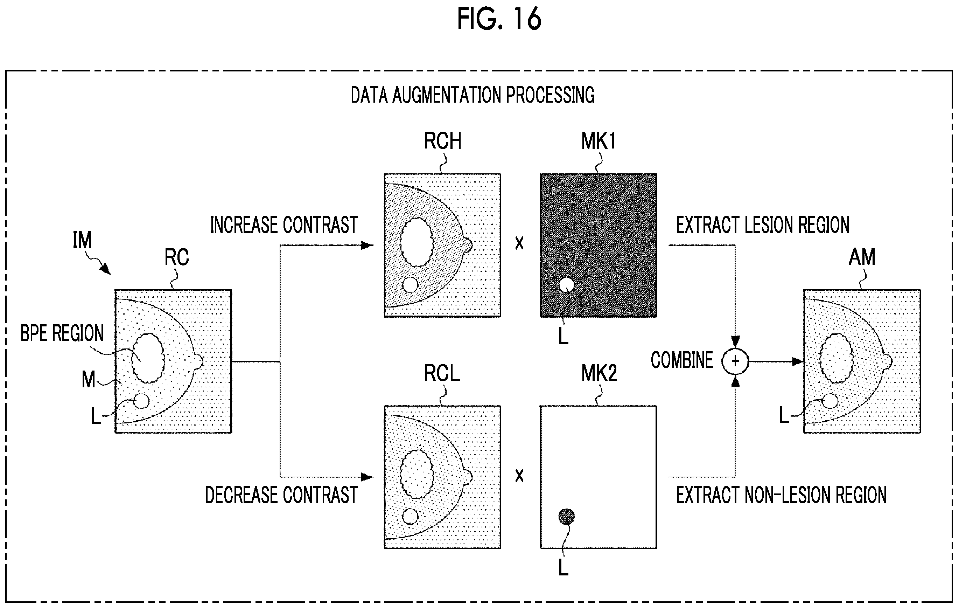

Fig. 16 conceptually shows the data augmentation processing. In the data augmentation processing, for example, a high-contrast image RCH obtained by increasing the contrast of the difference image RC and a low-contrast image RCL obtained by decreasing the contrast of the difference image RC are created. Next, the lesion region is extracted from the high-contrast image RCH by applying mask information MK1 for specifying the lesion region to the high-contrast image RCH. In addition, the non-lesion region is extracted from the low-contrast image RCL by applying mask information MK2 for specifying the non-lesion region to the low-contrast image RCL. The extracted lesion region and non-lesion region are combined to generate the augmented image AM in which the contrast between the lesion region and the non-lesion region is changed. Further, the low-energy image LE may be subjected to the data augmentation processing.

In the contrast-enhanced imaging, after the contrast agent is injected into the breast M, the contrast between the lesion region and the non-lesion region is changed. For example, since the contrast agent is more likely to be washed out in the BPE region, which is the non-lesion region, than in the lesion region, the contrast between the lesion and the BPE region is changed in accordance with the elapsed time after the contrast agent injection. That is, the contrast between the lesion region and the non-lesion region is changed in accordance with an imaging timing after the contrast agent is injected. Therefore, by performing the above-described data augmentation processing, the machine learning model 90 having high robustness against the variation in the imaging timing can be generated.

In order to increase the robustness, it is preferable to train the machine learning model 90 by generating a plurality of augmented images AM having different contrasts between the lesion region and the non-lesion region from one input image IM.

The machine learning model 90 that has been trained through the machine learning in the training phase is stored in the storage unit 42 as the MLM 60. It should be noted that the machine learning of the machine learning model 90 may be performed by the information processing apparatus 12 or may be performed by an external apparatus.

It should be noted that the MLM 60 may be generated by training the machine learning model 90 through the machine learning using only the low-energy image LE, and then retraining the machine learning model 90 using the difference image RC and the low-energy image LE.

### [Second Embodiment]

Hereinafter, the second embodiment will be described. In the above-described embodiment, the mammography apparatus 10 irradiates the breast M with the radiation R at one angle to acquire the radiation image. In the present embodiment, the mammography apparatus 10 enables tomosynthesis imaging that acquires the series of a plurality of radiation images by irradiating the breast M with the radiation at a plurality of angles.

In the present embodiment, the mammography apparatus 10 acquires the low-energy image LE and the high-energy image HE by irradiating the breast M in which the contrast agent is injected, with the radiation R having different energies for each angle. That is, a low-energy image group LEG consisting of a plurality of low-energy images LE and a high-energy image group HEG consisting of a plurality of high-energy images HE are generated by the tomosynthesis imaging.

In the present embodiment, the difference image generation unit 52 generates the difference image RC representing the difference between the low-energy image LE and the high-energy image HE for each angle. Therefore, a difference image group RCG consisting of a plurality of difference images RC is generated.

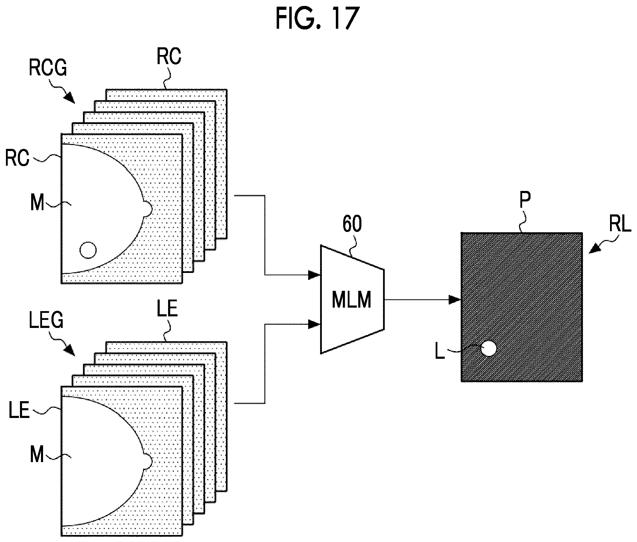

Fig. 17 schematically shows the lesion detection processing via the lesion detection processing unit 53 according to the second embodiment. In the present embodiment, the lesion detection processing unit 53 performs the lesion detection processing by inputting the difference image group RCG and the low-energy image group LEG to the MLM 60. It is preferable that the lesion detection processing unit 53 combines the difference image group RCG and the low-energy image group LEG in the channel direction to input the combined image group to the MLM 60. In addition, the lesion detection processing unit 53 need only input at least one of the low-energy image group LEG or the high-energy image group HEG and the difference image group RCG to the MLM 60. Other kinds of processing in the present embodiment are the same as in the first embodiment.

In the present embodiment, the low-energy image LE and the high-energy image HE are acquired by irradiating the breast M with the radiation R having different energies for each angle, so that the misregistration of the breast M between the low-energy image LE and the high-energy image HE acquired at the same angle is suppressed. As a result, it is possible to generate the difference image group RCG with high reliability, and it is possible to detect the lesion with high accuracy.

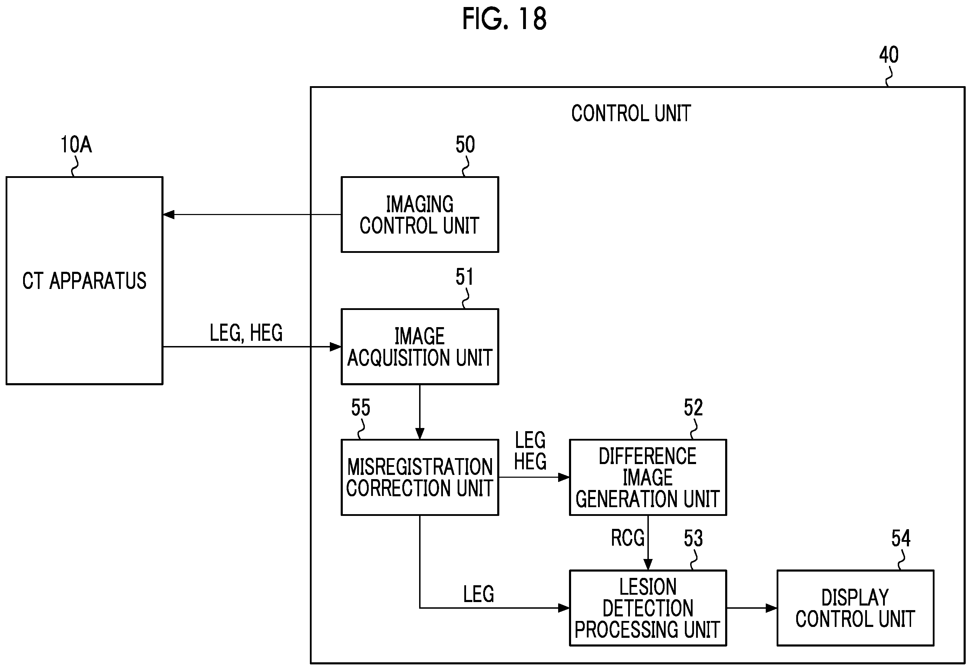

As shown in Fig. 18, as a modification example of the second embodiment, instead of the mammography apparatus 10, a computed tomography (CT) apparatus 10A can be used. The CT apparatus 10A captures a plurality of radiation images while rotating a pair of radiation sources and radiation detectors around the subject in which the contrast agent is injected. The CT apparatus 10A according to the present embodiment enables so-called dual-energy CT that performs the irradiation of the subject with the radiation having the first energy and the irradiation of the subject with the radiation having the second energy higher than the first energy. For example, 360° scanning around the subject is performed twice with the first energy and the second energy. As a result, as in a case of the tomosynthesis imaging, the low-energy image group LEG and the high-energy image group HEG are generated.

In the dual-energy CT, the energy of the radiation is changed for each scanning, and thus it is considered that an amount of misregistration of the subject between the low-energy image LE and the high-energy image HE acquired at the same angle is larger than that in a case of the tomosynthesis imaging. Therefore, as shown in Fig. 18, it is preferable that a misregistration correction unit 55 is provided in the latter part of the image acquisition unit 51. The misregistration correction unit 55 corrects the misregistration of the subject between the low-energy image group LEG and the high-energy image group HEG. The misregistration correction unit 55 supplies the low-energy image group LEG and the high-energy image group HEG in which the misregistration is corrected to the difference image generation unit 52, and supplies the low-energy image group LEG to the lesion detection processing unit 53. Other kinds of processing are the same as in the first embodiment.

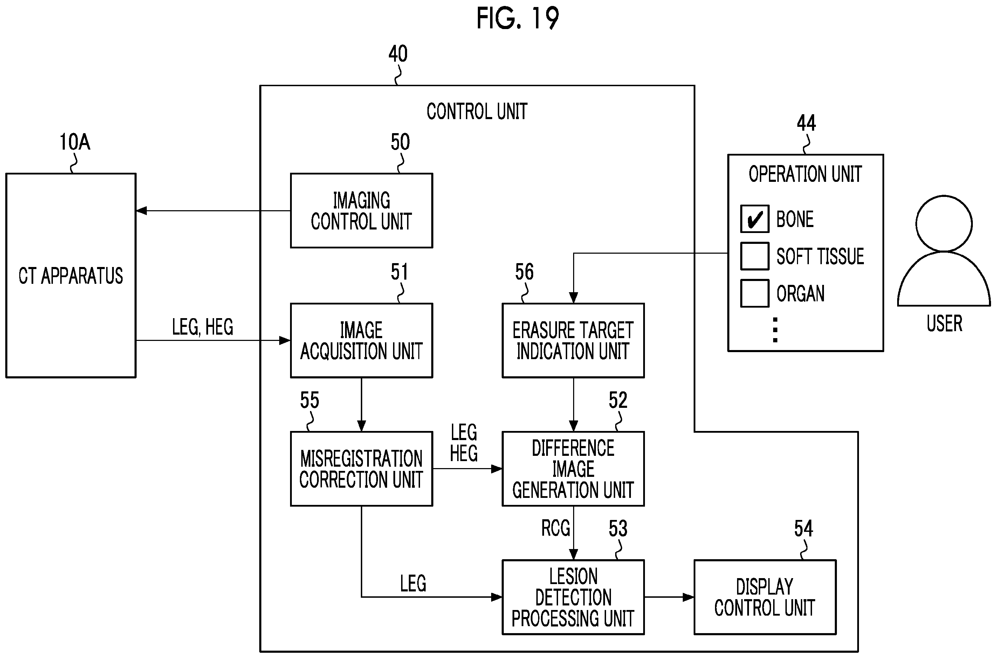

In addition, in the mammography, since the breast is the subject, almost all normal tissues are erased by performing energy subtraction to erase the mammary gland structure, but, in the CT, since a human body or the like is a subject, normal tissues having different compositions are present. Therefore, as shown in Fig. 19, it is preferable to provide an erasure target indication unit 56 that indicates a composition as an erasure target with respect to the difference image generation unit 52.

In the present embodiment, the user can designate the composition as the erasure target by using the operation unit 44. The composition that can be designated as the erasure target is, for example, a bone, a soft tissue, an organ, or the like. The erasure target indication unit 56 acquires information on the erasure target designated by using the operation unit 44, and indicates the erasure target with respect to the difference image generation unit 52. The difference image generation unit 52 erases the composition as the erasure target from the difference image RC by changing the first weight coefficient multiplied by the low-energy image LE and the second weight coefficient multiplied by the high-energy image HE, depending on the erasure target.

Further, instead of the CT apparatus 10A, a magnetic resonance imaging (MRI) apparatus can also be used. The CT apparatus 10A images a moisture content in cells, tissues, and the like in the subject by using the magnetic field. In the MRI, as in the CT, it is possible to perform the energy subtraction by using the contrast agent. In this case, at the high magnetic field, the subject is irradiated with the electromagnetic waves having first energy and the energy electromagnetic waves having second energy higher than the first energy, and the low-energy image group LEG and the high-energy image group HEG are generated.

### [Other Modification Examples]

The control unit 40 may accumulate information related to the contrast-enhanced imaging, information on the person under an examination on which the contrast-enhanced imaging is performed, and the like in the storage unit 42 or the like as data. For example, the control unit 40 may accumulate information such as an injection start time point of the contrast agent into the breast M, an imaging time point of the contrast-enhanced imaging (or an elapsed time from the start of the injection of the contrast agent to the imaging time point), a thickness of the breast M, imaging conditions (a tube voltage and the like), and other patient information (age, menstrual cycle, presence or absence of menopause, and the like) in the storage unit 42 or the like.

In addition, in each of the above-described embodiments and each of the above-described modification examples, as a hardware structure of a processing unit that executes various kinds of processing, such as the imaging control unit 50, the image acquisition unit 51, the difference image generation unit 52, the lesion detection processing unit 53, the display control unit 54, the misregistration correction unit 55, and the erasure target indication unit 56, various processors shown later can be used.

The various processors include a graphics processing unit (GPU) as well as a CPU. Further, the various processors include, in addition to a general-purpose processor which executes software (program) and functions as various processing units, such as a CPU, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application-specific integrated circuit (ASIC).

One processing unit may be configured by one of the various processors or may be configured by combining two or more processors of the same type or different types (for example, by combining a plurality of FPGAs or combining a CPU and an FPGA). Further, a plurality of the processing units may be configured by one processor.

A first example of the configuration in which the plurality of processing units are configured by one processor is a form in which one processor is configured by combining one or more CPUs and the software and this processor functions as the plurality of processing units, as represented by computers such as a client and a server. A second example is a form of using a processor that implements the function of the entire system including the plurality of processing units via one integrated circuit (IC) chip, as represented by a system on a chip (SoC) or the like. In this way, as the hardware structure, the various processing units are configured by using one or more of the various processors described above.

Further, the hardware structure of the various processors is, more specifically, an electric circuit (circuitry) in which circuit elements such as semiconductor elements are combined.

In addition, in the above-described embodiment and respective modification examples, the aspect has been described in which the program 42A is stored in the storage unit 42 in advance, but the present disclosure is not limited to this. The program 42A may be provided in a form of being recorded in a non-transitory recording medium such as a compact disc read only memory (CD-ROM), a digital versatile disc read only memory (DVD-ROM), or a universal serial bus (USB) memory. Further, the program 42A may be downloaded from an external apparatus via a network.

The embodiments and the respective modification examples can be combined as appropriate as long as there is no contradiction.

The above-described contents and the above-shown contents are detailed descriptions of portions related to the present disclosed technology and are merely examples of the present disclosed technology. For example, the description of the configuration, the function, the operation, and the effect are the description of examples of the configuration, the function, the operation, and the effect of the parts according to the present disclosed technology. Therefore, it goes without saying that unnecessary parts may be deleted, new elements may be added, or replacements may be made with respect to the above-described contents and the above-shown contents within a range that does not deviate from the gist of the present disclosed technology. Further, the description of, for example, common technical knowledge that does not need to be particularly described to enable the implementation of the present disclosed technology is omitted in the above-described contents and the above-shown contents in order to avoid confusion and to facilitate the understanding of the portions related to the present disclosed technology.

All of the documents, the patent applications, and the technical standards described in the present specification are incorporated into the present specification by reference to the same extent as in a case in which the individual documents, patent applications, and technical standards are specifically and individually stated to be described by reference.

The following technology can be understood from the above description.

### [Supplementary Note 1]

An information processing apparatus comprising: at least one processor, in which the processor is configured to: generate a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and detect a lesion based on at least any one of the low-energy image or the high-energy image and the difference image.

### [Supplementary Note 2]

The information processing apparatus according to supplementary note 1, in which the processor is configured to: detect the lesion by combining the difference image and the low-energy image in a channel direction and inputting the combined image to a machine learned model.

### [Supplementary Note 3]

The information processing apparatus according to supplementary note 2, in which the machine learned model includes a channel direction attention mechanism that performs weighting on each channel.

### [Supplementary Note 4]

The information processing apparatus according to supplementary note 1, in which the processor is configured to: detect the lesion based on a first feature value extracted from the difference image and a second feature value extracted from the low-energy image.

### [Supplementary Note 5]

The information processing apparatus according to supplementary note 4, in which the machine learned model includes a first pre-stage operation block, a second pre-stage operation block, and a post-stage operation block, and the processor is configured to: extract the first feature value by inputting the difference image to the first pre-stage operation block; extract the second feature value by inputting the low-energy image to the second pre-stage operation block; and detect the lesion by combining the first feature value and the second feature value in a channel direction and inputting the combined feature value to the post-stage operation block.

### [Supplementary Note 6]

The information processing apparatus according to supplementary note 5, in which the machine learned model includes a channel direction attention mechanism that performs weighting on each channel.

### [Supplementary Note 7]

The information processing apparatus according to any one of supplementary notes 1 to 6, in which the subject is a breast, and the electromagnetic waves are radiation.

### [Supplementary Note 8]

The information processing apparatus according to supplementary note 1, in which the subject is left and right breasts, the low-energy image includes a first low-energy image and a second low-energy image that are captured by irradiating each of the left and right breasts with radiation having the first energy, the high-energy image includes a first high-energy image and a second high-energy image that are captured by irradiating each of the left and right breasts with radiation having the second energy, and the difference image includes a first difference image representing a difference between the first low-energy image and the first high-energy image and a second difference image representing a difference between the second low-energy image and the second high-energy image.

### [Supplementary Note 9]

The information processing apparatus according to supplementary note 8, in which a first machine learned model includes a first pre-stage operation block and a first post-stage operation block, a second machine learned model includes a second pre-stage operation block and a second post-stage operation block, and the processor is configured to: extract a first feature value by combining the first difference image and the first low-energy image in a channel direction and inputting the combined image to the first pre-stage operation block; extract a second feature value by combining the second difference image and the second low-energy image in the channel direction and inputting the combined image to the second pre-stage operation block; detect the lesion in one of the left breast or the right breast by combining the second feature value with the first feature value and inputting the combined feature value to the first post-stage operation block; and detect the lesion in the other of the left breast or the right breast by combining the first feature value with the second feature value and inputting the combined feature value to the second post-stage operation block.

### [Supplementary Note 10]

The information processing apparatus according to supplementary note 9, in which the processor is configured to: combine the first feature value and the second feature value in the channel direction.

### [Supplementary Note 11]

The information processing apparatus according to supplementary note 10, in which the processor is configured to: combine the first feature value and the second feature value via a cross attention mechanism that generates a weight map representing a degree of relevance between the first feature value and the second feature value and that performs weighting on the first feature value and the second feature value based on the generated weight map.

### [Supplementary Note 12]

The information processing apparatus according to any one of supplementary note 2, 3, 5, or 6, in which the machine learned model is generated by training a machine learning model using training data including an input image and a ground-truth image and an augmented image in which a contrast between a lesion region and a non-lesion region in the input image is changed.

## Claims

1. An information processing apparatus comprising:
at least one processor,
wherein the processor is configured to:
generate a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and
detect a lesion based on at least any one of the low-energy image or the high-energy image and the difference image.

2. The information processing apparatus according to claim 1,
wherein the processor is configured to:
detect the lesion by combining the difference image and the low-energy image in a channel direction and inputting the combined image to a machine learned model.

3. The information processing apparatus according to claim 2,
wherein the machine learned model includes a channel direction attention mechanism that performs weighting on each channel.

4. The information processing apparatus according to claim 1,
wherein the processor is configured to:
detect the lesion based on a first feature value extracted from the difference image and a second feature value extracted from the low-energy image.

5. The information processing apparatus according to claim 4,
wherein the machine learned model includes a first pre-stage operation block, a second pre-stage operation block, and a post-stage operation block, and
the processor is configured to:
extract the first feature value by inputting the difference image to the first pre-stage operation block;
extract the second feature value by inputting the low-energy image to the second pre-stage operation block; and
detect the lesion by combining the first feature value and the second feature value in a channel direction and inputting the combined feature value to the post-stage operation block.

6. The information processing apparatus according to claim 5,
wherein the machine learned model includes a channel direction attention mechanism that performs weighting on each channel.

7. The information processing apparatus according to claim 1,
wherein the subject is a breast, and
the electromagnetic waves are radiation.

8. The information processing apparatus according to claim 1,
wherein the subject is left and right breasts,
the low-energy image includes a first low-energy image and a second low-energy image that are captured by irradiating each of the left and right breasts with radiation having the first energy,
the high-energy image includes a first high-energy image and a second high-energy image that are captured by irradiating each of the left and right breasts with radiation having the second energy, and
the difference image includes a first difference image representing a difference between the first low-energy image and the first high-energy image and a second difference image representing a difference between the second low-energy image and the second high-energy image.

9. The information processing apparatus according to claim 8,
wherein a first machine learned model includes a first pre-stage operation block and a first post-stage operation block,
a second machine learned model includes a second pre-stage operation block and a second post-stage operation block, and
the processor is configured to:
extract a first feature value by combining the first difference image and the first low-energy image in a channel direction and inputting the combined image to the first pre-stage operation block;
extract a second feature value by combining the second difference image and the second low-energy image in the channel direction and inputting the combined image to the second pre-stage operation block;
detect the lesion in one of the left breast or the right breast by combining the second feature value with the first feature value and inputting the combined feature value to the first post-stage operation block; and
detect the lesion in the other of the left breast or the right breast by combining the first feature value with the second feature value and inputting the combined feature value to the second post-stage operation block.

10. The information processing apparatus according to claim 9,
wherein the processor is configured to:
combine the first feature value and the second feature value in the channel direction.

11. The information processing apparatus according to claim 10,
wherein the processor is configured to:
combine the first feature value and the second feature value via a cross attention mechanism that generates a weight map representing a degree of relevance between the first feature value and the second feature value and that performs weighting on the first feature value and the second feature value based on the generated weight map.

12. The information processing apparatus according to claim 2,
wherein the machine learned model is generated by training a machine learning model using training data including an input image and a ground-truth image and an augmented image in which a contrast between a lesion region and a non-lesion region in the input image is changed.

13. An information processing method comprising:
generating a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and
detecting a lesion based on at least any one of the low-energy image or the high-energy image and the difference image.

14. A program causing a computer to execute a process comprising:
generating a difference image representing a difference between a low-energy image captured by irradiating a subject, in which a contrast agent is injected, with electromagnetic waves having first energy and a high-energy image captured by irradiating the subject with electromagnetic waves having second energy higher than the first energy; and
detecting a lesion based on at least any one of the low-energy image or the high-energy image and the difference image.
